(19) Europäisches Patentamt
European Patent Office
Office européen des brevets

(11) EP 4 692 053 A1

(12) **EUROPEAN PATENT APPLICATION**
published in accordance with Art. 153(4) EPC

(43) Date of publication:
**11.02.2026 Bulletin 2026/07**

(21) Application number: **24779850.7**

(22) Date of filing: **21.03.2024**

(51) International Patent Classification (IPC):
***C07C 321/04*** *(2006.01)* ***C07C 319/08*** *(2006.01)*
***C08G 18/38*** *(2006.01)* ***G02B 1/04*** *(2006.01)*
***G02C 7/00*** *(2006.01)*

(52) Cooperative Patent Classification (CPC):
**C07C 319/08; C07C 321/04; C08G 18/38; G02B 1/04; G02C 7/00**

(86) International application number:
**PCT/JP2024/011044**

(87) International publication number:
**WO 2024/203730 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 JP 2023058706**

(71) Applicant: **Hoya Lens Thailand Ltd.**
**Pathumthani 12130 (TH)**

(72) Inventors:
- **NAGASAWA, Takumi**
  **Tokyo 160-8347 (JP)**
- **KAWAKAMI, Hironori**
  **Tokyo 160-8347 (JP)**

(74) Representative: **Kraus & Lederer PartGmbB**
**Thomas-Wimmer-Ring 15**
**80539 München (DE)**

(54) **POLYTHIOL, POLYMERIZABLE COMPOSITION FOR OPTICAL MATERIALS, OPTICAL MATERIAL, AND METHODS FOR PRODUCING THOSE PRODUCTS**

(57) Provided is a polythiol obtained through a thiolation of a hydroxy group of a plant-derived polyol.



Processed by Luminess, 75001 PARIS (FR)

## Description

### Technical Field

The present invention relates to a polythiol, a polymerizable composition for optical materials, an optical material, and a method for producing the same.

### Background Art

PTL 1 discloses a polymerizable composition for optical materials containing a polyisocyanate, a polyol that is a plant-derived compound, and the like.

### Citation List

### Patent Literature

PTL 1: Japanese Patent No. 6571968

### Summary of Invention

### Technical Problem

It is desirable from the viewpoint of non-fossil resource utilization to produce optical materials using plant-derived raw materials. However, according to the studies by the present inventors, optical materials produced from the polymerizable composition for optical materials disclosed in PTL 1 have high haze, and further improvements in optical properties are required.
One aspect of the present invention has an object to provide a plant-derived compound suitable for preparing an optical material with excellent optical properties.

### Solution to Problem

As a result of intensive studies, the present inventors have newly found that a low-haze optical material can be produced by using a polythiol obtained by thiolating a hydroxy group (OH) of a plant-derived polyol to convert into a thiol group (SH).
That is, one aspect of the present invention is as follows.

[1] A polythiol obtained through a thiolation of a hydroxy group of a plant-derived polyol.
[2] The polythiol according to [1], wherein the plant-derived polyol is glycerol, sorbitol, isosorbide, sucrose, $\alpha$-methylglycoside, mannitol, 1,4-butanediol, 1,3-butanediol, or 1,3-propanediol.
[3] A polymerizable composition for optical materials, including: at least one polythiol as in [1] or [2]; and at least one polyiso(thio)cyanate.
[4] The polymerizable composition for optical materials according to [3], further including a plant-derived polyol.
[5] An optical material that is a cured product of the polymerizable composition for optical components as in [3].
[6] The optical material according to [5], which is a lens.
[7] The optical material according to [6], which is a spectacle lens.
[8] An optical material that is a cured product of the polymerizable composition for optical components as in [4].
[9] The optical material according to [8], which is a lens.
[10] The optical material according to [9], which is a spectacle lens.
[11] A method for producing a polythiol including thiolating a hydroxy group of a plant-derived polyol.
[12] A method for producing a polymerizable composition for optical materials, including:

producing a polythiol by the production method as in [11]; and
mixing the produced polythiol with at least one polyiso(thio)cyanate.

[13] A method for producing an optical material, including:

producing a polymerizable composition for optical materials by the production method as in [12]; and
curing the produced polymerizable composition for optical materials to prepare a cured product.

[14] The method for producing the optical material according to [13], wherein the optical material is a lens.
[15] The method for producing an optical material according to [14], wherein the lens is a spectacle lens.

Advantageous Effects of Invention

One aspect of the present invention provides a plant-derived compound suitable for preparing an optical material with excellent optical properties.

Description of Embodiments

[Polythiol and Method for Producing Same]

One aspect of the present invention relates to a polythiol obtained by thiolating hydroxy groups of a plant-derived polyol.
Another aspect of the present invention relates to a method for producing a polythiol, including thiolating a hydroxy group of a plant-derived polyol.
In the present invention and the present description, the term "plant-derived polyol" shall mean a polyol obtained from plants through single or multi-stage steps. Examples of the above steps may include a fermentation step, an extraction step, a separation step, a reaction step, and the like. The fact that a polyol includes carbon-$^{14}C$ means that the polyol is a plant-derived polyol. The fact that a polyol is plant-based can be confirmed by conducting measurements in accordance with ASTM D6866 METHOD B standard and detecting carbon-$^{14}C$. The same is true for the term "plant-derived" for the various compounds described below. In contrast, the term "non-plant-derived" described below means that a compound was obtained from a raw material other than plants, and the term "petroleum-derived" means that a compound was obtained from a petroleum raw material.
Plant-derived polyols can be obtained by known methods. Thiolation of a hydroxy group (OH) of the plant-derived polyol may be carried out, for example, by any of the following methods (1) to (4).

(1) A polyol is reacted with a hydrogen halide (for example, hydrobromic acid HBr, hydrochloric acid HCl, and the like) and thiourea to form an isothiuronium salt, and isothiuronium salt is hydrolyzed.
(2) A polyol is reacted with a hydrogen halide (for example, hydrobromic acid HBr) to form a halide (for example, a bromide), and then the resulting halide is reacted with a sulfide such as sodium sulfide, sodium hydrogen sulfide, potassium sulfide, and hydrogen sulfide.
(3) Hydrogen sulfide is reacted with a polyol at high temperature and high pressure in the presence of a catalyst.
(4) A polyol is reacted with methanesulfonyl chloride to yield a sulfonate. Alternatively, a polyol is reacted with tosyl chloride or acetic anhydride to yield a tosylate or an ester. A thioacetate salt (for example, potassium thioacetate) is reacted with the product thus obtained to yield a thioester, and the thioester is reduced with a reducing agent (for example, $LiAlH_4$). Alternatively, instead of the reducing agent, an acid (for example, HCl) may be added to hydrolyze the thioester.

The plant-derived polyol in which the hydroxy group is thiolated to be converted into a thiol group can be, for example, a polyol having 2 or more and 10 or less functional groups. The functional number of a polyol is the number of hydroxy groups contained in one molecule of the polyol. In a polythiol obtained through a thiolation of the hydroxy groups of a plant-derived polyol, all of the hydroxy groups of the plant-derived polyol may be thiolated, and some hydroxy groups may remain. Specific examples of plant-derived polyols may include glycerol, sorbitol, isosorbide, sucrose, $\alpha$-methylglycoside, mannitol, 1,4-butanediol, 1,3-butanediol, and 1,3-propanediol.
The biomass degree, which is an indicator for plant-derived raw materials, is measured according to the ASTM D6866 METHOD B standard and can be determined by the following equation. The biomass degree of the polythiol obtained through a thiolation of hydroxy groups of the plant-derived polyol can be, for example, 100%.

Biomass degree = Plant-derived carbon number / (Plant-derived carbon number + Non-plant-derived carbon number) $\times$ 100

[Polymerizable Composition for Optical Materials and Method for Producing Same]

One aspect of the present invention relates to a polymerizable composition for optical materials containing at least one polythiol obtained through a thiolation of hydroxy groups of a plant-derived polyol and at least one polyiso(thio)cyanate.
Also, one aspect of the present invention relates to a method for producing a polymerizable composition for

optical materials, including producing a polythiol by the above production method and mixing the produced polythiol with at least one polyiso(thio)cyanate. One or two or more polythiols produced by the above production method are mixed with one or two or more polyiso(thio)cyanates.

According to the polymerizable composition for optical materials, a polythiourethane-based optical material can be produced by the reaction between a polythiol and a polyiso(thio)cyanate.

In the present invention and the present description, the term "polyiso(thio)cyanate" is used to encompass both a polyisocyanate and a polyisothiocyanate. An isocyanate may also be called an isocyanate, and an isothiocyanate may also be called an isothiocyanate. Also, the term "iso(thio)cyanato group" shall be used to encompass both an isocyanato group (-N=C=O) and an isothiocyanato group (-N=C=S). The term "polyiso(thio)cyanate" is a polyfunctional compound having two or more iso(thio)cyanato groups in one molecule. By the reaction of a polythiol and a polyiso(thio)cyanate, thiol groups of the polythiol are reacted with iso(thio)cyanato groups of the polyiso(thio)cyanate, and a reaction product having, in a molecule, the following bond:

[C1]

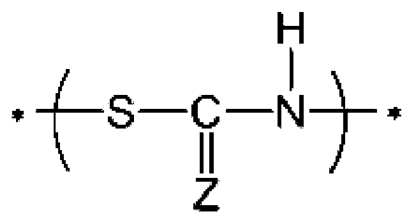

can be obtained. In the above formula, Z represents an oxygen atom or a sulfur atom. * represents the bonding position with adjacent structures. The reaction of a thiol group with an isocyanato group forms the above bond in which the X is an oxygen atom, and the reaction with an isothiocyanato group forms the above bond in which X is a sulfur atom. In the present invention and the present description, a reaction product (resin) containing a plurality of the above bonds in one molecule is described as "polythiourethane".

Polyiso(thio)cyanate may be a plant-derived polyiso(thio)cyanate or non-plant-derived (for example, petroleum-derived) polyiso(thio) cyanate. As the polyiso(thio)cyanate, various polyiso(thio)cyanates, such as an aliphatic polyiso(thio)cyanate, an alicyclic polyiso(thio)cyanate, and an aromatic polyiso(thio)cyanate may be used. The number of iso(thio)cyanato groups included in one molecule of polyiso(thio)cyanate is 2 or more, preferably 2 to 4, and more preferably 2 or 3. Specific examples of polyiso(thio)cyanates may include various compounds exemplified in paragraph [0052] in Japanese patent No. 5319037 as polyiso(thio) cyanate compounds. Preferable examples of polyiso(thio) cyanates may include aliphatic polyisocyanates such as hexamethylenediisocyanate, 1,5-pentanediisocyanate, isophorone diisocyanate, bis(isocyanatomethyl)cyclohexane, dicyclohexylmethane diisocyanate, 2,5-bis(isocyanatomethyl)-bicyclo[2.2.1]heptane, 2,6-bis(isocyanatomethyl)-bicyclo[2.2.1]heptane, bis(4-isocyanatocyclohexyl)methane, 1,3-bis(isocyanatomethyl)cyclohexane, and 1,4-bis(isocyanatomethyl)cyclohexane; and aromatic polyisocyanates such as bis(isocyanatomethyl)benzene, m-xylylene diisocyanate, p-xylylene diisocyanate, 1,3-diisocyanatobenzene, tolylene diisocyanate, 2,4-diisocyanatotoluene, 2,6-diisocyanatotoluene, and 4,4'-methylenebis(phenyl isocyanate). Furthermore, halogen-substituted products, such as chlorine-substituted products and bromine-substituted products, alkylsubstituted products, alkoxy-substituted products, and nitro-substituted products, and prepolymer-type denatures with polyhydric alcohols, carbodiimide denatures, urea denatures, biuret denatures, dimerization or trimerization reaction products, and the like of the polyiso(thio)cyanate may also be used. These compounds may be used singly or in combination of two or more thereof.

In the present invention and the present description, the term "polymerizable composition for optical materials" means a polymerizable composition used in the production of optical materials, and the term "polymerizable composition" means a composition containing at least one polymerizable compound. The polymerizable composition for optical materials contains at least one polythiol obtained through a thiolation of hydroxy groups of a plant-derived polyol, and at least one polyiso(thio)cyanate, and may or may not contain at least one other polymerizable compound. In the present invention and the present description, the term "polymerizable compound" means a compound that can undergo a polymerization reaction, and polythiols and polyiso(thio)cyanates are polymerizable compounds. Specific examples of other polymerizable compounds may include polyols. Specific examples of other polymerizable compounds may include polythiols other than polythiols obtained through a thiolation of hydroxy groups of plant-derived polyols.

When the polymerizable composition for optical materials contains a polyol as another polymerizable compound, such polyols may include plant-derived polyols or non-plant-derived (for example, petroleum-derived) polyols, and a plant-derived polyol is preferable. Specific examples of plant-derived polyols may include glycerol, sorbitol, isosorbide, sucrose, α-methylglycoside, mannitol, 1,4-butanediol, 1,3-butanediol, and 1,3-propanediol.

When the polymerizable composition for optical material contains, as other polymerizable compounds, a polythiol other than polythiols obtained through a thiolation of hydroxy groups of a plant-derived polyol, examples of such polythiols may include non-plant-derived (for example, petroleum-derived) polythiols. Specific examples of non-plant-derived (for example, petroleum-derived) polythiols may include a mixture of 4,7-bis(mercaptomethyl)-3,6,9-trithiaundecane-1,11-dithiol, 4,8-bis (mercaptomethyl)-3,6,9-trithiaundecane-1,11-dithiol, and 5,7-bis(mercaptomethyl)-3,6,9-trithiaundecane-1,11-dithiol; 4-mercaptomethyl-1,8-dimercapto-3,6-dithioaoctane, pentaerythritol tetrakis(3-mercaptopropionate), pentaerythritol tetrakis(2-mercaptoacetate), and the like. However, non-plant-derived polyols are not limited to the above specific examples. A non-plant-derived (for example, petroleum-derived) polythiol can be synthesized in a known method. For synthetic methods, refer to Japanese Patent No. 3444682, Japanese Patent No. 6632493, Japanese Patent No. 6588639, Japanese Patent No. 4928543, and Japanese Patent No. 6851763, and Japanese Patent Application Publication No. S63-46213, Japanese Patent Application Publication No. S63-46213, Japanese Patent Application Publication No. 2005-336104, and Japanese Patent Application Publication No. S59-152367, and the like, for example.

The above-described polymerizable composition for optical materials can be prepared by mixing the various components described above. The mixing proportion between a polythiol obtained through a thiolation of hydroxy groups of a plant-derived polyol in the polymerizable composition for optical materials and polyiso(thio)cyanate is not particularly limited and, for example, as molar ratio, (thiol groups included in a polythiol obtained through a thiolation of hydroxy groups of a plant-derived polyol) / (iso(thio)cyanato groups included in a polyisoi(thio)cyanate) is within the range of 0.5 to 3.0, preferably 0.6 to 2.0, and still more preferably 0.8 to 1.3. In one embodiment, the polymerizable composition for optical materials contains a polythiol obtained through a thiolation of hydroxy groups of a plant-derived polyol in an amount of, for example, 10 mass% or more (for example,10 to 50 mass%) in relation to 100 mass% of the total amount of the polymerizable composition for optical materials.

When the polymerizable composition for optical materials contains a polyol, the content of the polyol may be, for example, 10 mass% or less (for example, 1 to 10 mass%) in relation to 100 mass% of the total amount of the polymerizable composition for optical materials.

When the polymerizable composition for optical materials contains a polythiol other than polythiols obtained through a thiolation of hydroxy groups of a plant-derived polyol, the content of the polyol may be, for example, 20 mass% or less (for example, 1 to 20 mass%) in relation to 100 mass% of the total of the polymerizable composition for optical materials.

When preparing the polymerizable composition for optical materials, at least one other component other than the various components described above may be mixed. Specific examples of such other components may include a reaction catalyst for the reaction between a polythiol and a polyiso(thio)cyanate. For other components that may be mixed, refer to paragraphs [0055], [0057], and [0058] to [0064] of Japanese Patent No. 5319037, for example. In addition, at least one common additive commercially available as an additive for various resins, such as a polythiourethane resin, may be used. The polymerizable composition for optical materials may be prepared by mixing various components described above simultaneously or sequentially in any order. The preparation method is not particularly limited, and any known method for preparing a polymerizable composition can be adopted without any limitation.

[Optical Material and Method for Producing Same]

One aspect of the present invention relates to an optical material that is a cured product obtained by curing the polymerizable composition for optical materials.

Another aspect of the present invention relates to a method for producing an optical material, the method including: producing a polymerizable composition for optical materials by the production method described above; and curing the produced polymerizable composition for optical materials to yield a cured product.

The optical material may be a lens such as a spectacle lens. The curing reaction of the polymerizable composition for optical materials can be performed using various curing treatments capable of curing the polymerizable composition. For example, to produce a cured product (also referred to as a "plastic lens") having a lens shape, cast polymerization is preferable. In cast polymerization, a cured product can be obtained by injecting a curable composition into a cavity of a molding die having two molds facing each other with a predetermined gap therebetween and the cavity formed by closing the gap, and polymerizing (curing) the curable composition inside the cavity. For details on molding dies that can be used for cast polymerization, refer to paragraphs [0012] to [0014] and Fig. 1 in Japanese Patent Application Publication No. 2009-262480, for example. The above publication shows a molding die in which the gap between the two molds is closed with a gasket as a sealing member, but a tape can also be used as the sealing member.

In one form, the cast polymerization can be performed as follows. The polymerizable composition is injected into the cavity of the molding die from an injection port provided on the side surface of the molding die. After injection, the polymerizable composition can be polymerized (cured), preferably by heating, to cure the polymerizable composition, thereby producing a cured product to which the internal shape of the cavity is transferred. The polymerization conditions

are not particularly limited, and may be set, as appropriate, depending on the composition of the polymerizable composition and the like. As an example, a molding die in which a polymerizable composition has been injected into a cavity can be heated at a heating temperature of 10°C to 150°C for 1 to 72 hours, although the conditions for polymerization are not limited to these. In the present invention and the present description, the temperature, such as the heating temperature, for the cast polymerization refers to the atmospheric temperature (for example, the temperature in the furnace) at which the molding die is disposed. Also, during heating, the temperature can be raised at any temperature-raising rate and lowered (cooled) at any temperature-dropping rate. After the completion of the polymerization (curing reaction), the cured product inside the cavity is detached from the molding die. The cured material can be detached from the molding die by removing the top and bottom molds that form the cavity and a gasket or a tape in any order, as is normally done in cast polymerization. The cured product detached from the molding die can preferably be used as a spectacle lens (in particular, a lens substrate). The cured product to be used as the lens substrate for spectacle lenses usually can be subjected to post-processes after the detachment from the molding die, for example, annealing, a grinding step such as a rounding step, a polishing step, and a coat layer forming step of forming a primer coat layer for improving impact resistance, a hard coat layer for increasing the surface hardness, and the like. Furthermore, various functional layers, such as an antireflection layer and a water-repellent layer, can be formed on the lens substrate. For any of these steps, known techniques can be used without any limitation. Thus, a spectacle lens in which the lens substrate is the cured product can be obtained. Further, by attaching this spectacle lens to the frame, glasses can be obtained.

The biomass degree of the optical material can be, for example, 20% or more. Also, the biomass degree of the optical material may be 100% and can be 100% or less, 90% or less, 80% or less, 70% or less, 60% or less, or 50% or less. An optical material with a high biomass degree is desirable from the viewpoint of non-fossil resource utilization. The biomass degree can be determined by a known method. Specific examples of the way of calculating the biomass degree of the optical material may include the methods described in the example section below.

In one embodiment, the optical material can exhibit a high refractive index. Regarding the refractive index of the optical material, for example, the refractive index ne can be 1.50 or more or 1.55 or more. The optical material may have a refractive index ne of 1.76 or less. The refractive index ne is the refractive index at a wavelength of 546.1 nm.

## Examples

Hereinafter, the present invention will be further described with reference to Examples, but the present invention is not limited to the embodiments shown in the Examples. The operations and evaluations described below were performed at room temperature (about 20°C to 25°C) in the air unless otherwise noted.

### [Example 1]

(1) Thiolation of Hydroxy Groups of Plant-Derived Polyol

To 92.1 g (1.0 mol) of plant-derived glycerol, 100 mL of pure water, 570.9 g (7.5 mol) of thiourea, and 911.4 g (9.0 mol) of 36 mass% hydrochloric acid were added, and the resulting mixture was stirred for 9 hours under reflux at 110°C. After cooling to room temperature, 400 mL of toluene was added, then 1801.2 g (13.5 mol) of a 30 mass% aqueous sodium hydroxide solution was added gradually, and hydrolysis was performed at 60°C for 4 hours. The resulting organic layer was sequentially washed twice with 100 mL of 36 mass% hydrochloric acid and then with 100 mL of water, and toluene was removed in a rotary evaporator to yield a crude product. Subsequently, impurities are removed by single distillation to yield 80.0 g (0.57 mol, 99% purity) of a target polythiol compound (trithioglycerol; hereinafter referred to as "TTG").

Measurements were performed in accordance with the ASTM D6866 METHOD B standard and the biomass degree was determined using the following equation to find 100%.

Biomass degree = Plant-derived carbon number / (Plant-derived carbon number + Non-plant-derived carbon number) $\times$ 100

(2) Preparation of Lens

First, 64.8 parts by mass of a petroleum-derived xylylene diisocyanate (hereinafter referred to as "XDI"), 0.01 parts by mass of dimethyltin dichloride as a curing catalyst, 0.20 parts by mass of acidic phosphate ester "JP-506H" (manufactured by Johoku Chemical Co., Ltd.) as a release agent, and 0.5 parts by mass of a UV-absorbing agent "SEESORB 707" (manufactured by Shipro Kasei Kaisha Ltd.) were mixed and dissolved. Then, 35.2 parts by mass of TTG obtained in the above (1) were added and mixed to prepare a mixed homogeneous solution.

This mixed homogeneous solution was defoamed for 1 hour at 200 Pa and then filtered through a 5.0 μm PTFE (polytetrafluoroethylene) filter.

Next, the solution was then poured into a molding die for lenses made of a glass mold with a diameter of 75 mm and -4.00 D and 0.00 D and a tape.

The molding die was placed in an electric furnace, gradually heated from 15°C to 100°C over 20 hours, and then maintained at 100°C for 2 hours to facilitate polymerization. After the polymerization reaction was completed, the molding die was removed from the electric furnace, and the lens was detached therefrom to prepare a lens. The resulting lens was further annealed for 3 hours in an annealing furnace having a furnace temperature of 100°C.

[Example 2]

A lens was prepared in the same manner as above, except that the XDI of Example 1 was replaced with a mixture (hereinafter, referred to as "petroleum-derived NBDI") of petroleum-derived 2,5-bis(isocyanatomethyl)-bicyclo[2.2.1]heptane and petroleum-derived 2,6-bis(isocyanatomethyl)-bicyclo[2.2.1]heptane.

[Example 3]

(1) Thiolation of Hydroxy Groups of Plant-Derived Polyol

A reaction vessel was charged with 50 g (342 mmol) of plant-derived isosorbide and 500 mL of dry dichloromethane. Thereafter, the resulting mixture was cooled to 0°C, then 48 mL (342 mmol) of triethylamine was added, and 27.2 mL (419 mmol) of methanesulfonic acid was added dropwise over 15 minutes to the solution. Thereafter, the mixture was then warmed to room temperature and stirred for 2 hours. The organic layer was washed, water content was removed, then the solvent was removed under reduced pressure to yield 80 g (265 mmol) of reaction intermediate A. Subsequently, 80 g (265 mmol) of the reaction intermediate A and 500 mL of dimethylacetamide (DMA) were added and stirred at room temperature for 30 min. Then, 60 g (530 mmol) of potassium thioacetate was added, and the resulting mixture was stirred at 120°C for 3 hours. The organic layer was washed, water content was then removed, and the solvent was removed under reduced pressure to yield 44 g (168 mmol) of a reaction intermediate B.

To a 1 L flask, 44 g (168 mmol) of the reaction intermediate B and 500 mL of anhydrous THF were added, and the resulting mixture was stirred for 30 minutes at room temperature, nitrogen gas was filled, the reaction mixture was cooled to 0°C, 7.4 g (168 mmol) of $LiAlH_4$ was added, and the resulting mixture was stirred for 1 hour. The reaction was diluted with tetrahydrofuran (THF), dehydrated with sodium sulfate, and the supernatant was concentrated under reduced pressure to yield the crude product. Thereafter, the single distillation was repeated twice to remove impurities, and the objective 1,4:3,6-dianhydro-2,5-dithio-D-mannitol (hereinafter, referred to as "ISH"), 12 g (67 mmol, 99% purity, liquid at room temperature), was obtained. The biomass degree was determined in the same manner as in Example 1, and the biomass degree was 100%.

(2) Preparation of Lens

In 60.5 parts by mass of petroleum-derived XDI, 0.01 parts by mass of dimethyltin dichloride, 0.20 parts by mass of "JP-506H" (manufactured by Johoku Chemical Industry Co., Ltd.), and 0.5 parts by weight of "SEESORB 707" (manufactured by Shipro Kasei Kaisha, Ltd.) were mixed and dissolved. Further, 19.6 parts by mass of TTG obtained in (1) of Example 1 and 20.0 parts by mass of ISH obtained in (1) of Example 3 were added and mixed to prepare a mixed homogeneous solution. Thereafter, a lens was prepared in the same manner as in Example 1.

[Example 4]

A lens was prepared in the same manner as in Example 3, except that XDI was replaced with petroleum-derived NBDI.

[Example 5]

A lens was prepared in the same manner as in Example 3, except that TTG was replaced with petroleum-derived polythiol 1 (4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol).

[Example 6]

A lens was prepared in the same manner as in Example 4, except that TTG was replaced with petroleum-derived polythiol 1 synthesized in a known manner.

[Example 7]

In 66.9 parts by mass of petroleum-derived XDI, 0.01 parts by mass of dimethyltin dichloride, 0.20 parts by mass of "JP-506H" (manufactured by Johoku Chemical Industry Co., Ltd.), and 0.5 parts by mass of "SEESORB 707" (manufactured by Shipro Kasei Kaisha, Ltd.) were mixed and dissolved. Further, 27.8 parts by mass of TTG obtained in (1) of Example 1 and 5.3 parts by mass of plant-derived 1,3-butanediol were added and mixed, and stirring was continued until a clear homogeneous solution was obtained. Thereafter, a lens was prepared in the same manner as in Example 1.

[Example 8]

A lens was prepared in the same manner as in Example 7, except that XDI was replaced with petroleum-derived NBDI.

[Comparative Example 1]

To 75.8 parts by mass of petroleum-derived XDI, 0.01 parts by mass of dimethyltin dichloride, 0.20 parts by mass of "JP-506H" (manufactured by Johoku Chemical Industry Co., Ltd.)), and 0.5 parts by weight of "SEESORB 707" (manufactured by Shipro Kasei Kaisha, Ltd.) were mixed and dissolved. Thereafter, when 24.2 parts by mass of the plant-derived glycerol was added and mixed, the cloudy mixed solution generated heat and gelated, so that the lens could not be prepared.

[Comparative Example 2]

Lens preparation was attempted in the same manner as in Comparative Example 1, except that XDI was replaced with petroleum-derived NBDI, but the mixed solution gelled as in Comparative Example 1, and the lenses could not be prepared.

[Comparative Example 3]

In 65.6 parts by mass of petroleum-derived XDI, 0.01 parts by mass of dimethyltin dichloride, 0.20 parts by mass of "JP-506H" (manufactured by Johoku Chemical Industry Co., Ltd.), and 0.5 parts by weight of "SEESORB 707" (manufactured by Shipro Kasei Kaisha, Ltd.) were mixed and dissolved. Thereafter, when 23.0 parts by mass of plant-derived isosorbide and 11.5 parts by weight of glycerol were added and mixed, isosorbide, which was solid, was not dissolved in the mixture solution, and a part of the mixture solution was gelated. Therefore, no lens could be prepared.

[Comparative Example 4]

Lens preparation was attempted in the same manner as in Comparative Example 1, except that XDI was replaced with petroleum-derived NBDI, but, as in Comparative Example 3, isosorbide was not dissolved in the mixture solution, and a part of the mixture solution was gelated. Therefore, no lens could be prepared.

Monomer ratios (molar ratios) of the respective polymerizable compositions of Examples 1 to 8 and Comparative Examples 1 to 4 are listed in Table 1. Although petroleum-derived polyisocyanate was used as the polyisocyanate in this case, plant-derived polyisocyanate may also be used.

[Table 1]

| | Monomer ratios (Molar ratios) | | | | | | | |
|---|---|---|---|---|---|---|---|---|
| | Polyisocyanate | | Polythiol | | | Polyol | | |
| | Petroleum-derived | | OH in plant-derived polyol is converted into SH | | Petroleum -derived | Plant-derived | | |
| | XDI | NBDI | TTG | ISH | Polythiol 1 | 1,3-Butanediol | Glycerol | Isosorbide |
| Example1 | 0.60 | - | 0.40 | - | - | - | - | - |
| Example2 | - | 0.60 | 0.40 | - | - | - | - | - |

(continued)

| | Monomer ratios (Molar ratios) | | | | | | | |
|---|---|---|---|---|---|---|---|---|
| | Polyisocyanate | | Polythiol | | | Polyol | | |
| | Petroleum-derived | | OH in plant-derived polyol is converted into SH | | Petroleum -derived | Plant-derived | | |
| | XDI | NBDI | TTG | ISH | Polythiol 1 | 1,3-Butanediol | Glycerol | Isosorbide |
| Example3 | 0.56 | - | 0. 24 | 0.20 | - | - | - | - |
| Example4 | - | 0.56 | 0. 24 | 0.20 | - | - | - | - |
| Example5 | 0.57 | - | - | 0.29 | 0.14 | - | - | - |
| Example6 | - | 0.57 | - | 0.29 | 0.14 | - | - | - |
| Example7 | 0.58 | - | 0. 32 | - | - | 0.10 | - | - |
| Example8 | - | 0.58 | 0. 32 | - | - | 0.10 | - | - |
| Compar. Example 1 | 0.60 | - | - | - | - | - | 0.40 | - |
| Compar. Example2 | - | 0.60 | - | - | - | - | 0.40 | - |
| Compar. Example3 | 0.55 | - | - | - | - | - | 0. 20 | 0.25 |
| Compar. Example4 | - | 0.55 | - | - | - | - | 0.20 | 0. 25 |

[Comparative Example 5]

A lens was prepared in the same procedure as in Example A1 of Japanese Patent No. 6571968 (PTL 1).

[Comparative Example 6]

A lens was prepared in the same procedure as in Example A6 of Japanese Patent No. 6571968 (PTL 1).

On the respective lenses of Examples 1 to 8 and Comparative Examples 5 and 6, the biomass degrees were determined from the prescription of the polymerizable compositions used to prepare a lens according to the following equation:

Biomass degree = {[Parts by mass of (b) × Biomass degree of (b)/100] + [Parts by mass of (c) × Biomass degree of (c)/100]}/ [Total mass (parts by mass) of (a) + (b) + (c) + (d)]

The (a) to (d) in the above equation are as follows.

(a): Petroleum-derived polyisocyanate
(b): Polythiol obtained through a thiolation of hydroxy groups of a plant-derived alcohol
(c): Plant-derived polyol
(d): Petroleum-derived polythiol

The biomass degree in (c) was measured according to the ASTM D6866 METHOD B standard and determined by the following equation. Any plant-derived polyol had a biomass degree of 100%.

Biomass degree = Plant-derived carbon number / (Plant-derived carbon number + Non-plant-derived carbon number) × 100

[Evaluation Method]

<Refractive Index ne>

The refractive indexes ne of the respective lenses of Examples 1 to 8 and Comparative Examples 5 and 6 were measured using a precision refractometer KPR-2000, manufactured by Kalnew Optical Industrial Co., Ltd., at a measurement temperature of 23°C.

<Striae>

For the respective lenses of Examples 1 to 8 and Comparative Examples 5 and 6, a projection inspection was performed using an appearance inspection device (Optical Modulex SX-UI251HQ manufactured by Ushio Inc.). Using a highpressure UV lamp (USH-102D manufactured by Ushio Inc.) as a light source, a white screen was installed at a distance of 1 m from the light source, a lens to be evaluated was inserted between the light source and the screen, and the projected image on the screen was observed, and evaluation was performed according to the following criteria.

(Evaluation Criteria)

A: There were no linear irregularities in the projected image.
B: There were thin linear irregularities in the projected image.
C: There were linear thick irregularities in the projected image.
D: There were significant linear irregularities in the projected image.

<Haze Value>

Haze values of the respective lenses of Examples 1 to 8 and Comparative Examples 5 and 6 were measured using a haze meter (MH-150 manufactured by Murakami Color Research Laboratory).

The above results are shown in Table 2.

[Table 2]

| | Refractive index ne | Biomass degree | Striae | Haze value |
|---|---|---|---|---|
| Example1 | 1.653 | 33 | A | Less than 0.3 |
| Example2 | 1.622 | 31 | A | Less than 0.3 |
| Example3 | 1. 637 | 40 | A | Less than 0.3 |
| Example4 | 1. 608 | 38 | A | Less than 0.3 |
| Example5 | 1.635 | 20 | A | Less than 0.3 |
| Example6 | 1. 606 | 18 | A | Less than 0.3 |
| Example7 | 1.631 | 33 | C | 0.3 or more and less than 0.5 |
| Example8 | 1.602 | 31 | B | 0.3 or more and less than 0.5 |
| Compar. Example1 | No lens could be prepared due to gelation during mixing step | | | |
| Compar. Example2 | No lens could be prepared due to gelation during mixing step | | | |
| Compar. Example3 | No lens could be prepared because isosorbide was not dissolved | | | |
| Compar. Example4 | No lens could be prepared because isosorbide was not dissolved | | | |
| Compar. Example5 | 1.51 | 26 | D | 0.5 or more |
| Compar. Example6 | 1.52 | 28 | D | 0.5 or more |

The results shown in Table 2 show that haze generation is suppressed in the lenses of Examples 1 to 8 compared to the lenses of Comparative Examples 5 and 6, and that the generation of striae is also suppressed. Furthermore, it can also be confirmed that the lenses of Examples 1 to 8 are lenses with higher refractive indexes compared to those of the lenses of Comparative Examples 5 and 6.

Two or more of the various aspects and the various embodiments described in the present specification can be

combined together in any combination.

The embodiments disclosed herein are only examples in all respects and should not be considered as restrictive. The scope of the present invention is not limited to the above description, but is defined by the scope of claims, and is intended to encompass equivalents to the scope of claims and all modifications within the scope of the claims.

Industrial Applicability

One aspect of the present invention is useful in the technical field of various kinds of optical materials.

## Claims

1. A polythiol obtained through a thiolation of a hydroxy group of a plant-derived polyol.

2. The polythiol according to claim 1, wherein the plant-derived polyol is glycerol, sorbitol, isosorbide, sucrose, α-methylglycoside, mannitol, 1,4-butanediol, 1,3-butanediol, or 1,3-propanediol.

3. A polymerizable composition for optical materials, comprising: at least one polythiol as in claim 1 or 2; and at least one polyiso(thio)cyanate.

4. The polymerizable composition for optical materials according to claim 3, further comprising a plant-derived polyol.

5. An optical material that is a cured product of the polymerizable composition for optical components as in claim 3.

6. The optical material according to claim 5, which is a lens.

7. The optical material according to claim 6, which is a spectacle lens.

8. An optical material that is a cured product of the polymerizable composition for optical components as in claim 4.

9. The optical material according to claim 8, which is a lens.

10. The optical material according to claim 9, which is a spectacle lens.

11. A method for producing a polythiol, comprising thiolating a hydroxy group of a plant-derived polyol.

12. A method for producing a polymerizable composition for optical materials, comprising:

    producing a polythiol by the production method as in claim 11; and
    mixing the produced polythiol with at least one polyiso(thio)cyanate.

13. A method for producing an optical material, comprising:

    producing a polymerizable composition for optical materials by the production method as in claim 12; and
    curing the produced polymerizable composition for optical materials to prepare a cured product.

14. The method for producing an optical material according to claim 13,
wherein the optical material is a lens.

15. The method for producing an optical material according to claim 14,
wherein the lens is a spectacle lens.

## INTERNATIONAL SEARCH REPORT

International application No.
**PCT/JP2024/011044**

**A. CLASSIFICATION OF SUBJECT MATTER**

***C07C 321/04***(2006.01)i; ***C07C 319/08***(2006.01)i; ***C08G 18/38***(2006.01)i; ***G02B 1/04***(2006.01)i; ***G02C 7/00***(2006.01)i
FI: C07C321/04; C08G18/38 076; C07C319/08; G02C7/00; G02B1/04

According to International Patent Classification (IPC) or to both national classification and IPC

**B. FIELDS SEARCHED**

Minimum documentation searched (classification system followed by classification symbols)

C07C321/04; C07C319/08; C08G18/38; G02B1/04; G02C7/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2015/046370 A1 (MITSUI CHEMICALS, INC.) 02 April 2015 (2015-04-02) claims, manufacturing examples, examples, in particular, manufacturing example 3, example 21 | 1, 3-15 |
| X | CN 102086363 A (XIAMEN UNIVERSITY) 08 June 2011 (2011-06-08) paragraphs [0013]-[0023], examples, claims | 1-2, 11 |
| Y | | 1-2, 11 |
| Y | WO 2015/060260 A1 (MITSUI CHEMICALS, INC.) 30 April 2015 (2015-04-30) claims, paragraphs [0002], [0003], [0008] | 1-2, 11 |
| A | WO 2018/030391 A1 (HOYA LENS THAILAND LTD.) 15 February 2018 (2018-02-15) entire text, all drawings | 1-15 |
| A | WO 2016/052069 A1 (FUJIFILM CORPORATION) 07 April 2016 (2016-04-07) entire text, all drawings | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 May 2024** | **11 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/011044**

**C. DOCUMENTS CONSIDERED TO BE RELEVANT**

| Category* | Citation of document, with indication, where appropriate, of the relevant passages | Relevant to claim No. |
|---|---|---|
| A | JP 58-164615 A (TORAY INDUSTRIES, INC.) 29 September 1983 (1983-09-29) entire text, all drawings | 1-15 |

Form PCT/ISA/210 (second sheet) (July 2022)

**INTERNATIONAL SEARCH REPORT**
**Information on patent family members**

International application No.
**PCT/JP2024/011044**

| Patent document cited in search report | Publication date (day/month/year) | Patent family member(s) | Publication date (day/month/year) |
|---|---|---|---|
| WO 2015/046370 A1 | 02 April 2015 | US 2015/0342276 A1 claims, examples | |
| | | JP 2016-21040 A | |
| | | JP 2016-20549 A | |
| | | US 2015/0346387 A1 | |
| | | WO 2015/046369 A1 | |
| | | EP 3051331 A1 | |
| | | EP 3037411 A1 | |
| | | EP 3168256 A1 | |
| | | CN 105556377 A | |
| | | KR 10-2016-0044036 A | |
| | | KR 10-2015-0063573 A | |
| | | KR 10-2015-0063576 A | |
| | | CN 104781229 A | |
| | | CN 105017080 A | |
| CN 102086363 A | 08 June 2011 | (Family: none) | |
| WO 2015/060260 A1 | 30 April 2015 | US 2016/0237198 A1 claims | |
| | | JP 2016-47910 A | |
| | | JP 2016-47911 A | |
| | | US 2016/0282515 A1 | |
| | | WO 2015/060259 A1 | |
| | | EP 3061780 A1 | |
| | | EP 3061778 A1 | |
| | | KR 10-2016-0052619 A | |
| | | CN 105658694 A | |
| | | KR 10-2016-0052618 A | |
| | | CN 105658693 A | |
| WO 2018/030391 A1 | 15 February 2018 | US 2018/0297943 A1 entire text, all drawings | |
| | | EP 3381893 A1 | |
| | | CN 108473420 A | |
| WO 2016/052069 A1 | 07 April 2016 | CN 106573885 A | |
| JP 58-164615 A | 29 September 1983 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6571968 B
- JP 5319037 B
- JP 3444682 B
- JP 6632493 B
- JP 6588639 B
- JP 4928543 B
- JP 6851763 B
- JP S6346213 B
- JP 63046213 A
- JP 2005336104 A
- JP 59152367 A
- JP 2009262480 A